# EUROPÄISCHE PATENTANMELDUNG

(11) **EP 3 473 214 A1**
(43) Veröffentlichungstag der Anmeldung: **24.04.2019**
(21) Anmeldenummer: 17196977.7
(22) Anmeldetag: 18.10.2017
(51) Int. Cl.: A61F 2/958, A61B 17/22, A61B 17/3207, A61F 2/844

(54) **BALLONKATHETHER-STENT-VORRICHTUNG**

(71) Anmelder: Biotronik AG, 8180 Bülach (CH)
(72) Erfinder: Risch, Fabian, 8200 Schaffhausen (CH); Schäfer, Tobias, 78176 Blumberg-Fützen (DE)
(74) Vertreter: Galander, Marcus

(57) **Zusammenfassung**

Ballonkatheter-Stent-Vorrichtung, welche aufweist: einen Ballonkatheter, der einen Katheterschlauch und einen radial aufweitbaren Ballonabschnitt umfasst, und einen den Ballonabschnitt umgebenden Stent, der durch den und in seiner radialen Erstreckung im Wesentlichen korrespondierend zum Ballonabschnitt radial aufweitbar ist, wobei der Stent mindestens ein, bevorzugt eine Mehrzahl von Faltwerken umfasst, die sich allein in Folge einer Aufweitung des Ballons und somit des Stents in Folge der hiermit einhergehenden Verkürzung des Stents lokal über dessen globalen End-Umfang hinaus falten und aufstellen und äußere Stent-Vorsprünge bilden.

## Beschreibung

Die Erfindung betrifft eine Ballonkatheter-Stent-Vorrichtung, welche einen Ballonkatheter, der einen Katheterschlauch und einen radial aufweitbaren Ballonabschnitt umfasst, und einen den Ballonabschnitt umgebenden Stent aufweist, der durch den und in seiner radialen Erstreckung im Wesentlichen korrespondierend zum Ballonabschnitt radial aufweitbar ist.

Derartige Vorrichtungen sind seit langem bekannt und hunderttausendfach im klinischen Einsatz zur Behandlung von Gefäßverengungen (Stenosen) von Blutgefäßen. Die seit langem bekannte Behandlung durch Aufweitung (Dilatation) mittels eines Ballonkatheters wird bei solchen Vorrichtungen ergänzt um eine Stabilisierung des aufgeweiteten Gefäßabschnitts mittels eines Stützelementes, das durch den mittels des Ballons aufgeweiteten und nach der Deflatation des Ballons und dem Zurückziehen des Ballonkatheters am Ort der Stenose zurückgelassenen Stent (Stenting). Zu derartigen Vorrichtungen wird rein beispielhaft auf die DE 10 2004 059 523 A1 hingewiesen, die eine neuere Entwicklungsstufe dieser Vorrichtungen beschreibt.

In vielen Fällen ist zusätzlich zur Aufweitung und Stabilisierung des Stenose-Abschnitts ein mindestens partielles Aufbrechen von Ablagerungen auf der Gefäßwand wünschenswert. Zu diesem Zweck wurden Ballonkatheter mit sogenanntem Schneidballon (Cutting Balloons) mit Schneidklingen auf der Ballonoberfläche sowie neuerdings auch Stents mit Werkzeugelementen (Scoring Elements) zur Bearbeitung von Gefäßablagerungen entwickelt und auch bereits klinisch eingesetzt. Die WO 2009/046206 A1 zeigt einen Ballonkatheter mit aufgesetzten Scoring-Elementen. Die US 8,348,987 B2 zeigt eine Ballonkatheter-Stent-Vorrichtung mit auf der Stentstruktur aufgesetzten Scoring-Elementen.

Es hat sich gezeigt, dass beide Lösungen erhebliche Nachteile haben.

Schneidballone sind in Folge der aufgesetzten Schneidelemente relativ steif und können nur schwer in komplexe Läsionen eingeführt werden, und es besteht hier auch ein nennenswertes Perforationsrisiko. Über dies ist der minimale (deflatierte) Ballondurchmesser größer als bei vergleichbaren Ballonen ohne Schneidelemente. Schließlich leisten Schneidballone beim Vorschieben der Vorrichtung durch das Gefäßsystem einen relativ großen Widerstand, der die präzise Handhabung erschwert.

Bei Stents mit Scoring-Elementen besteht unter Anderem die Gefahr, dass die auch im Einführungs-Zustand abstehenden Scoring-Elemente an unerwünschten und unvorhergesehenen Stellen zu Gewebeschädigungen führen. Andererseits sind die am beabsichtigten Einsatzort bei klinischen Studien tatsächlich erzielten Wirkungen nicht zufriedenstellend.

Der Erfindung liegt daher die Aufgabe zu Grunde, eine verbesserte Vorrichtung der genannten Art bereitzustellen, die zu einem Aufbrechen von Gefäßablagerungen im Zuge der Ballondilatation und Stent-Versorgung von Stenosen geeignet und leicht und sicher handhabbar ist.

Diese Aufgabe wird durch eine Ballonkatheter-Stent-Vorrichtung mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, die Vorrichtung mit mindestens einem Elemente, bevorzugt einer Vielzahl von Elementen, auszustatten, das sich im Ausgangszustand (deflatierten Zustand des Ballons) praktisch in die Oberfläche des Stents einfügt, jedoch im Gebrauchs- bzw. Endzustand (inflatierten Zustand des Ballons und dilatierten Zustand des Stents) aus der Oberfläche des Stents lokal hervorspringt und hierdurch eine Bearbeitungswirkung gegenüber den Stent im Gebrauchszustand umgebenden Materialschichten erzielen kann. Weiterhin schließt die Erfindung den Gedanken ein, diese nur im Gebrauchszustand vorhandenen Stent-Vorsprünge unter Nutzung der Verformung zu erzeugen, die der Stent bei der Dilatation erfährt. Schließlich gehört zur Erfindung der Gedanke, die Stent-Vorsprünge durch Faltwerke zu bilden, die sich im Ausgangs-Zustand im aufgefalteten bzw. ausgebreiteten Zustand befinden, und in die Oberfläche des Stents eingebettet sind während sie durch die Verkürzung des Stents bei seiner Aufweitung in den Falt-Zustand versetzt werden und sich somit über die globale End-Umfangsfläche des Stents hinaus lokal aufstellen.

Gemäß der Grundidee der Erfindung wird die Verkürzung des Stents, welche zwangsläufig mit der radialen Expansion durch die Inflation des Ballons erfolgt, für das Aufstellen der Faltwerke auszunutzen. Radial expandierbare Stents weisen in Umfangsrichtung eine irgendwie geartete Mäander- oder Zick-Zack- oder sonstige Wellenstruktur auf. Bei radialer Expansion werden diese Strukturen in Umfangsrichtung auseinander gezogen und gleichzeitig in axialer Richtung entsprechend gestaucht. Vereinfacht ausgedrückt vergrößert sich die Wellenlänge einer in Umfangsrichtung verlaufenden Struktur während sich ihre Amplitude verkleinert. Die in axialer Richtung verkürzend wirkenden Kräfte werden bei der vorliegenden Erfindung zum Falten und Aufstellen der Stent-Vorsprünge genutzt.

Mit der erfindungsgemäßen Lösung werden, zumindest in bevorzugten Ausführungen, unter anderem folgende Vorteile erzielt:
- Durch die Faltwerke wird eine große radiale Expansion erreicht. Das erlaubt einen sehr kleinen Querschnitt des Ballonkatheters. Somit können Stenosen erreicht werden, die sonst nicht oder nur mit einem mehrschrittigen Prozedur aufgeweitet werden können.
- Durch die Faltwerke und die Struktur ist das Aufstellen der Stent-Vorsprünge geführt und robust.
- Je nach Anwendungsfall (Aufbrechen von Stenosen oder Verankerung, von z. B. Herzklappen) sind flexible Ausführungen möglich.
- Vorteilhafter Transport und Zuführen des Implantats wegen kleinem Querschnitt und relativ ungestörter Oberfläche.

Grundsätzlich ist die vorgeschlagene Lösung und die Erzielung der genannten Vorteile auch in einer reinen Stent-Vorrichtung (ohne Ballonkatheter) realisierbar. Dann wird die gleiche Stent-Struktur nicht mittels des Ballons des Ballonkatheters, sondern auf andere Weise dilatiert, und die Dilatation realisiert die gleichen Funktionen und hat die gleichen Wirkungen wie oben beschrieben. Aus derzeitiger Sicht ist die Vorrichtung mit Ballonkatheter jedoch bevorzugt, weil sie größere tangentiale bzw. radiale Kräfte bereitstellen kann und somit eine grundsätzlich robustere und zuverlässige Ausführung der Erfindung ermöglichen dürfte.

In Ausführungen der Erfindung sind die Faltwerke derart ausgebildet, dass sie bei ihrer Faltung und Aufstellung Stent-Vorsprünge mit einer Höhe über der globalen End-Umfangsfläche des Stents ausbilden, die abhängig vom jeweiligen globalen End-Umfang des Stents ist. Dies ermöglicht gewissermaßen eine "selbsttätige" Einstellung der wirksamen Höhe der mit den Stent-Vorsprüngen geschaffenen Werkzeugabschnitte (bzw. Verankerungshilfen) an die Dimensionen des zu behandelnden Gefäßes bzw. der jeweiligen Läsion.

In bevorzugten Ausführungen der Erfindung sind die Faltwerke derart ausgebildet, dass bei ihrer zunehmenden Faltung und Aufstellung eine axiale und eine radiale und/oder tangentiale Bewegungskomponente auftritt. Hierdurch lässt sich bereits im Vorgang der Inflation des Ballons und Dilatation des Stents eine Relativbewegung zur umgebenden Stenose und somit eine gewisse Bearbeitungs-Wirkung erzielen, ohne dass es hier zu einer gesonderten Bewegung des Ballonkatheters bedürfte.

In einer weiteren Ausführung der Erfindung sind die Faltwerke derart ausgebildet, dass sie im Ausgangszustand des Stents aufgefaltet in dessen globaler Ausgangs-Umfangsfläche liegen oder sich nicht mehr als 20%, bevozugt 1% bis 10%, des Ausgangs-Radius aus dieser erheben. Dies ermöglicht die im Hinblick auf ein möglichst leichtes und gefahrloses Einführen der Vorrichtung wünschenswerte "ungestörte" Oberflächenkontur des undilatierten Stents. Bevorzugt liegen die Faltwerke im Ausgangszustand des Stents (und auch im gecrimpten Zustand bei der Implantation) in der Ausgangs-Umfangsfläche, d.h. die Faltwerke erstrecken sich nicht radial über die restliche Strebenstruktur des Stents hinaus. Faltwerke, die sich aus der Ausgangs-Umfangsfläche hinaus erheben, kommen zweckmäßigerweise nur in speziellen Fällen zum Einsatz. In diesen speziellen Fällen erheben sich die Faltwerke vorteilhafterweise nicht mehr als 20%, bevorzugt zwischen 1% und 10%, des Ausgangs-Radius aus der Ausgangs-Umfangsfläche hinaus.

Zweckmäßigerweise wird die Kombination aus dem Ballon und darauf befindlichen Stent mit Faltwerken auf einen Außendurchmesser zwischen 1.2 mm und 2.2 mm komprimiert, so dass die Kombination geeignet ist, über einen 4F bis 6F Zugang in den Körper eines Patienten eingeführt zu werden. Der Ballon und damit der Innendurchmesser des Stents mit Faltwerken kann vorteilhafterweise auf einen Durchmesser zwischen 2 mm und 10 mm expandiert werden. Die Faltwerke sorgen für eine Radiusvergrösserung von bis zu 1.7mm. Abhängig von Verhältnis zwischen komprimierten und expandierten Durchmesser des Stents verkürzt sich der Stent bei der Expansion um bis zu 15 mm. Bevorzugt sorgen die Faltwerke für eine Durchmesservergößerung im expandierten Zustand auf einen Wert zwischen 110% und 200%, bevorzugt zwischen 125% und 175% besonders bevorzugt zwischen 130% und 160% Hierbei entsprechen 100% dem Durchmesser des expandierten Stents ohne Faltwerke.

Die axiale Verkürzung treibt wie bereits erläutert das radiale Aufstellen der Faltwerke. In einer alternativen Ausgestaltung der Erfindung kann die axiale Verkürzung für eine zusätzliche relative Bewegung der Faltwerke gegenüber der Gefäßwand genutzt werden. In dieser Ausgestaltung wird das Stentdesign so gewählt, dass mit der Expansion eine extreme axiale Verkürzung um nahezu 100% Prozent der Stentlänge einhergeht. Dadurch werden die Faltwerke bei Expansion nicht nur aufgestellt sondern verändern auch ihre axiale Position bezüglich des Katheters, d.h. die Faltwerke bewegen sich nach distal oder proximal in Bezug auf die Katheterachse und damit auch zur behandelnden Gefäßwand. In dieser Ausgestaltung kann somit ein aktives Aufbrechen, ähnlich einem Aufschneiden, der Stenose kommen.

In weiteren Ausführungen der Erfindung haben die Faltwerke im gefalteten und aufgestellten Zustand im Wesentlichen eine Pyramiden- oder überlagerte Tetraeder-Form. Grundsätzlich sind vielgestaltige andere Ausführungen der Faltwerke möglich, sofern deren Aufbau nicht zu kompliziert und die gezielte Faltung unter Einsatzbedingungen zuverlässig sichergestellt ist. Die Wahl einer konkreten Geometrie wird sich auch nach dem Einsatzgebiet und der vorranging beabsichtigten Funktion der Stent-Vorsprünge (etwa Aufsprengen oder Abtragen von Stenosen oder Verankerung des Stents) richten.

In weiteren Ausführungen weist der Stent eine reguläre Gitterstruktur aus Gitterstabelementen auf, und die Faltwerke sind mindestens teilweise von Gitterstabelementen gebildet oder an diese fest angefügt. Stents, die aus regulären Gitterstrukturen gebildet sind, sind in vielgestaltigen Ausführungen bekannt und bewährt, und ihre Herstellung ist technologisch ausgereift und kostengünstig. Die genannte Ausführung der Erfindung kann auf diesen Vorteilen aufbauen und somit gleichfalls zu besonders zuverlässigen und kostengünstigen Produkten führen.

Im Übrigen können die Faltwerke integral mit der übrigen Struktur des Stents und insbesondere mittels des gleichen Formgebungsverfahrens wie jene gebildet sein, etwa durch im Wesentlichen einstufiges Laserschneiden der Gesamtstruktur aus einem rohrförmigen Halbzeug. Alternativ können die Faltwerke aus einem anderen Material als die übrige Struktur des Stents gebildet und in einem gesonderten Anfügungs-Schritt an jene angefügt sein. Letztere Varianten ermöglichen eine besonders differenzierte Ausführung der Faltwerke, bei gleichzeitiger Nutzung einer bewährten Stent-Grundstruktur.

In weiteren Ausführungen ist vorgesehen, dass Endabschnitte bzw. -punkte von Faltkanten der Faltwerke mit Querschnittsverjüngungen oder Perforationen als Soll-Biegestellen oder einem Formgedächtnis-Material gebildet sind. Der Ort, wo die Faltwerke gefaltet werden kann also vorgängig bestimmt werden durch Materialschwächungen oder durch Einprägung der Form (z. B. bei Nitinol). Durch entsprechende Verjüngungen und/oder durch den unter dem Faltwerk liegenden Ballon wird sichergestellt, dass sich das Faltwerk nach außen ausbreitet.

In aus derzeitiger Sicht bevorzugten Ausführungen, die direkt dem zu Grunde liegenden Problem gewidmet sind, weist mindestens ein Teil der Faltwerke jeweils mindestens eine Schneid- oder Sägekante zum schneidenden oder sägenden Eingriff in ein äußeres Material, insbesondere eine Stenose eines Blutgefäßes, auf. Die Funktion des durch die eine oder mehreren Schneid- oder Sägekante/n gebildeten Werkzeugs lässt sich durch die Geometrie des zu Grunde liegenden Faltwerks und die selektive Platzierung der jeweiligen Bearbeitungskante/n in weiten Grenzen variieren.

In anderen Ausführungen weist mindestens ein Teil der Faltwerke jeweils mindestens eine gebogene, insbesondere konkav gekrümmte, Fixierungskante zum Eingriff in eine äußere Struktur, insbesondere die Wandung eines Blutgefäßes oder Organs, auf.

In weiteren Ausführungen sind auf der Umfangsfläche des Stents mindestens in Zwischenbereichen zwischen einigen Faltwerken elastisch (oder ggfs. auch plastisch) verformbare Ausgleichsbereiche zur Verringerung der resultierenden Verkürzung des Stents vorgesehen. Je nach Anordnung können solche Federelemente auch eine Relativbewegung der Faltwerke zur Stenose bewirken, was für das Schneiden der Verkalkung von Vorteil wäre.

Die Faltwerke können auch Grundelemente sein, wo andere Komponenten angebracht sind z. B. ein Stab, der mit mehreren Faltwerken verbunden ist.

Die Stentstruktur kann lose auf dem Ballon liegen, fest mit ihm verbunden oder aber auch fest/lose mit dem Ballonhals oder Schlauch verbunden sein.

Im Falle eines Implantats, das durch die Faltwerke besser verankert werden soll, ist eine lose Verbindung zum Ballon/Schlauch zweckmäßig.
Für den bevorzugten Anwendungsfall des Aufbrechens eine Stenose, bei der die Scoring Elemente wieder rausgeführt werden müssen, ist eine zumindest teilweise feste Verbindung zum Ballon/Schlauch nötig.

Als Scoring-Vorrichtung ist es von Vorteil, wenn die Stentstruktur aus einem Formgedächtnis-Material (z. B. Nitinol) ist, damit sie sich nach erfolgtem Aufbrechen der Verkalkung wieder auf den ursprünglichen Querschnitt zurückzieht. Die im Formgedächtnis-Material eingeprägte Form der Stentstruktur ist dabei die ursprüngliche Form, wo die Faltwerke nicht aufgestellt sind. Die Kraft für das Aufstellen der Faltwerke kommt in diesem Fall von der Inflation eines Ballons. Wenn der Ballon deflatiert wird, geht die Stentstruktur von sich aus wieder in die eingeprägte Form zurück . Die Faltwerke falten sich somit wieder ein.

Andere Materialen sind aber auch denkbar, vor allem wenn die Struktur zur Verankerung dient. In diesem Fall ist eine Zurückfalten der Faltwerke nicht nötig. Aber auch beim Aufbrechen einer Stenose kommen Materialien für Stent und Faltwerke in Frage, die keine Formgedächtniseigenschaften aufweisen. In diesem Fall werden die Materialien plastisch durch die Inflation des Ballons verformt (die Faltwerke werden aufgerichtet) und durch die Kraft des deflatierenden Ballons wieder plastisch zurück verformt (die Faltwerke gehen wieder in den komprimierten Ausgangszustand zurück). In dieser Ausgestaltung der Verbindung sind Ballon und Stent fest verbunden, bevorzugt über die gesamte Auflagefläche des Stents.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1A und 1B: skizzenartige perspektivische Darstellungen des Grundaufbaus einer Ballonkatheter-Stent-Vorrichtung im Ausgangs-Zustand bzw. im dilatierten End-Zustand,
- Fig. 2: eine schematische Darstellung des Faltwerks einer Ausführungsform der erfindungsgemäßen Ballonkathether-Stent-Vorrichtung,
- Fig. 3: eine schematische Darstellung des Faltwerks einer Ausführungsform der erfindungsgemäßen Ballonkathether-Stent-Vorrichtung, und
- Fig. 4: eine schematische Zusammenstellung von Grund-Geometrien von Faltwerken erfindungsgemäßer Ballonkatheter-Stent-Vorrichtungen, jeweils in Draufsicht (links) und Seitenansicht (rechts) im dilatierten Zustand des Stents.

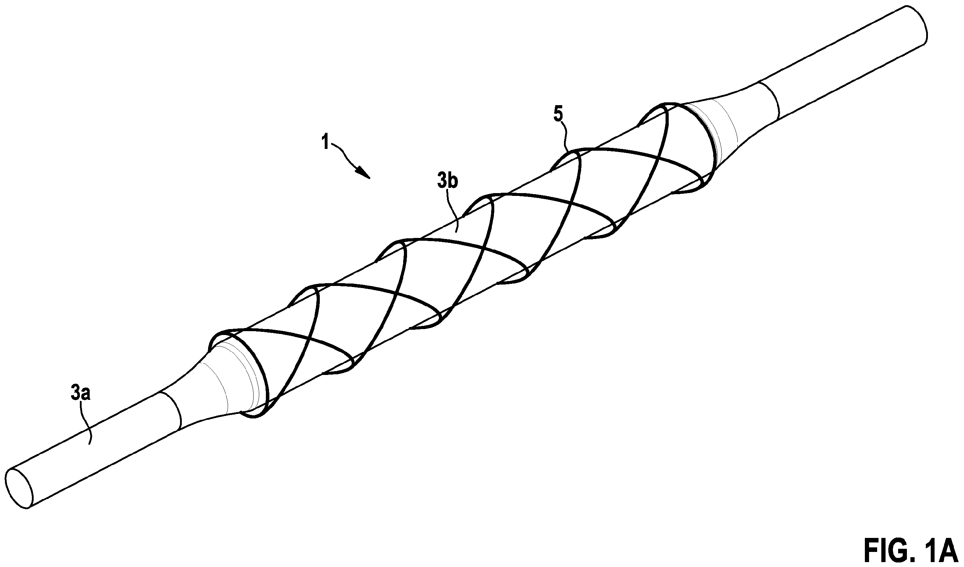

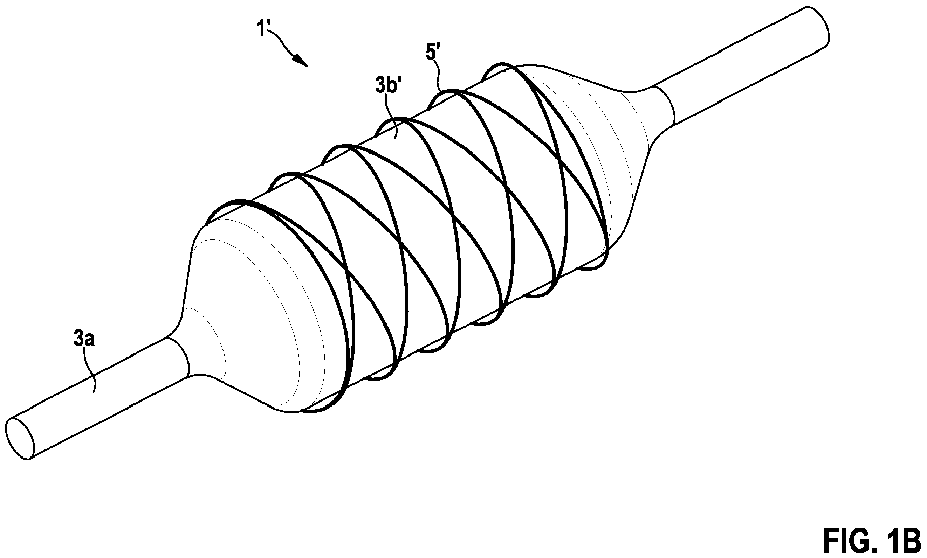

Fig. 1A zeigt schematisch eine Ballonkathether-Stent-Vorrichtung 1, die einen Ballonkatheter 3 mit einem Katheterschlauch 3a und einem Ballonabschnitt 3b und einen den Ballonabschnitt umgebenden Stent 5 aufweist, im nicht-inflatierten bzw. un-dilatierten Ausgangszustand, und Fig. 1B zeigt die dilatierte Ballonkatheter-Stent-Vorrichtung 1' mit inflatiertem Ballonabschnitt 3b' und Stent 5' in einem End-Zustand, wie er nach Einführung in ein Gefäßsystem durch Inflation des Ballonabschnitts zum Zwecke der Behandlung einer Stenose bewirkt wird. Der Stent 5' hat in bekannter Weise eine Gitterstruktur, die sich bei der Dilatation radial aufweitet und axial verkürzt.

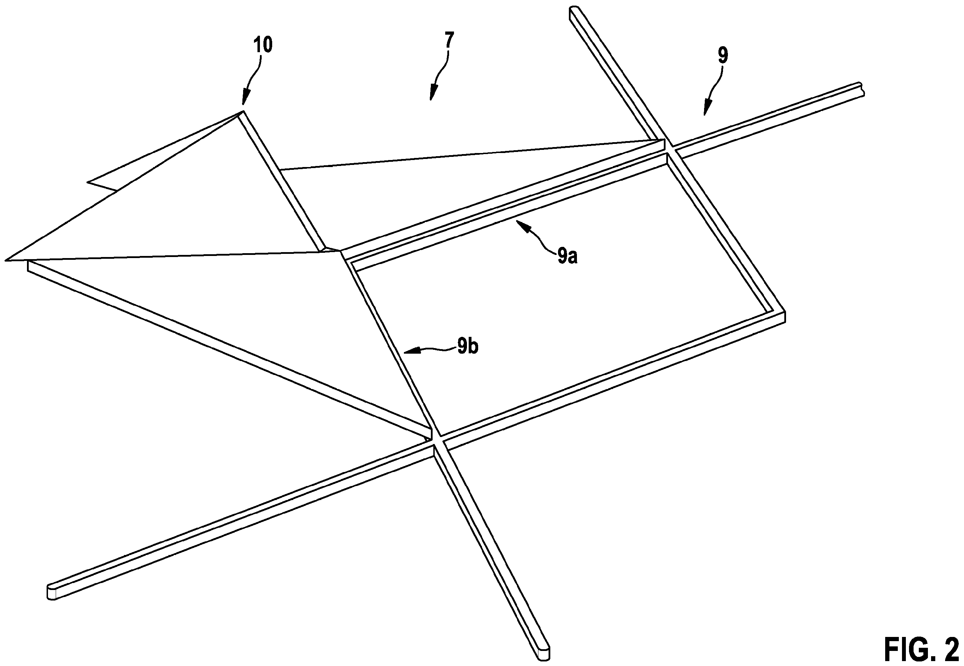

Fig. 2 zeigt in einer schematischen perspektivischen Darstellung ein Faltwerk 7, welches an zwei Gitterstabelementen 9a, 9b einer Gitterstruktur 9 eines Stents angebracht ist, in einem gefalteten und somit einen Vorsprung 10 über der übrigen Oberfläche ("globalen Umfangsfläche") des Stents bildenden End-Zustand. Dieser Zustand entspricht dem in Fig. 1B gezeigten dilatierten Zustand des Stents 5', bei dem die die Umfangsfläche bildende Gitterstruktur zwar radial expandiert, aber axial verkürzt ist. Die axiale Verkürzung bewirkt das Falten und Aufstellen der im Ausgangs-Zustand im Wesentlichen flach in der Umfangsfläche liegenden Faltwerkes 7.

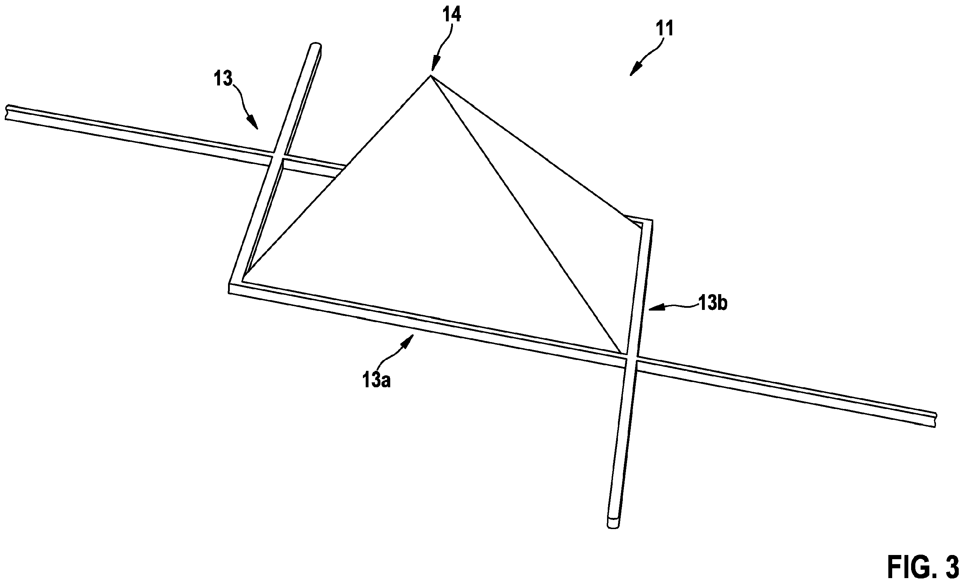

Fig. 3 zeigt als weitere Ausführungsform ein Faltwerk 11, welches in dem hier gezeigten End-Zustand die Faltungs-Geometrie eines einfachen Tetraeders hat, wiederum an zwei Gitterstabelementen 13a, 13b einer Stent-Gitterstruktur festgemacht ist und einen über die übrige Oberfläche der Stent-Gitterstruktur radial hinausragenden Vorsprung 14 bildet.

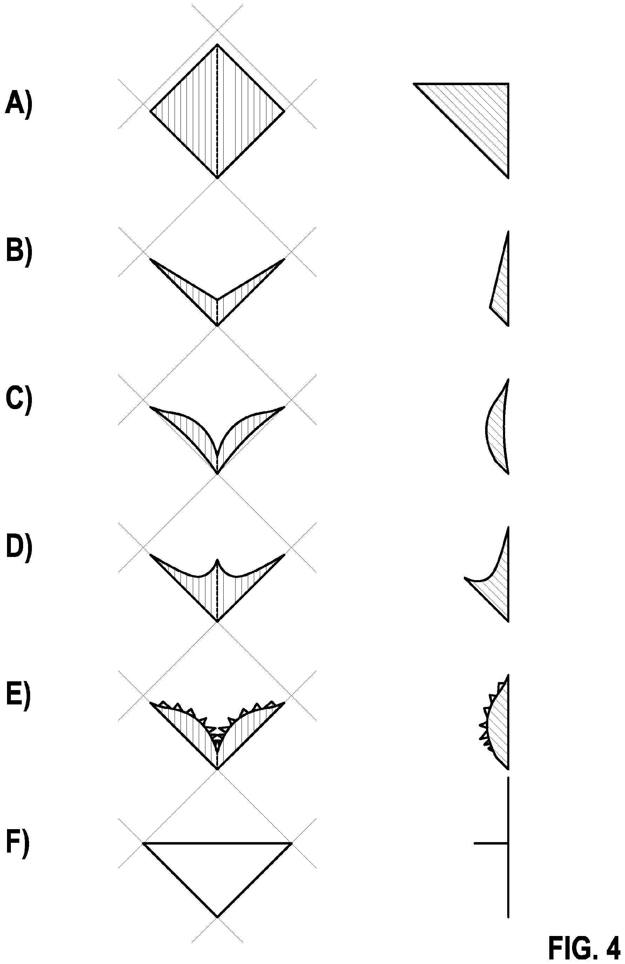

Fig. 4 zeigt skizzenhaft weitere geometrische Konfigurationen a) bis f) von Faltwerken im aufgestellten End-Zustand, wobei Vorsprünge (10 und 14) mit unterschiedlichen Winkeln (Fig. 4a und Fig 4b) ausgestaltet sind oder konvex (Fig. 4c) und konkav gekrümmte (Fig. 4d)oder mit Sägezahngeometrie versehene (Fig. 4e) Kanten ausgebildet sind. Fig. 4f zeigt eine Faltwerksgeometrie aus einzelnen Stabelementen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Ballonkatheter-Stent-Vorrichtung (1; 1'), welche aufweist:
einen Ballonkatheter (3; 3'), der einen Katheterschlauch (3a) und einen radial aufweitbaren Ballonabschnitt (3b; 3b') umfasst, und
einen den Ballonabschnitt umgebenden Stent (5; 5'), der durch den und in seiner radialen Erstreckung im Wesentlichen korrespondierend zum Ballonabschnitt radial aufweitbar ist,
wobei der Stent mindestens ein, bevorzugt eine Mehrzahl von, Faltwerk(en) (7; 11) umfasst, die sich allein in Folge einer Aufweitung des Ballons und somit des Stents in Folge der hiermit einhergehenden Verkürzung des Stents lokal über dessen globalen End-Umfang hinaus falten und aufstellen und äußere Stent-Vorsprünge (10; 14) bilden.

2. Ballonkatheter-Stent-Vorrichtung nach Anspruch 1, wobei die Faltwerke (7; 11) derart ausgebildet sind, dass sie bei ihrer Faltung und Aufstellung Stent-Vorsprünge (10; 14) mit einer Höhe über der globalen End-Umfangsfläche des Stents ausbilden, die abhängig vom jeweiligen globalen End-Umfang des Stents ist.

3. Ballonkatheter-Stent-Vorrichtung nach Anspruch 1 oder 2, wobei die Faltwerke (7; 11) derart ausgebildet sind, dass bei ihrer zunehmenden Faltung und Aufstellung eine axiale und eine radiale und/oder tangentiale Bewegungskomponente auftritt.

4. Ballonkatheter-Stent-Vorrichtung nach einem der vorangehenden Ansprüche, wobei die Faltwerke (7; 11) derart ausgebildet sind, dass sie im Ausgangszustand des Stents (5) aufgefaltet in dessen globaler Ausgangs-Umfangsfläche liegen oder sich nicht mehr als 20%, bevorzugt zwischen 1% und 10%, des Ausgangs-Radius aus dieser erheben.

5. Ballonkatheter-Stent-Vorrichtung nach einem der vorangehenden Ansprüche, wobei die Faltwerke (7; 11) im gefalteten und aufgestellten Zustand im Wesentlichen eine Pyramiden- oder überlagerte Tetraeder-Form haben.

6. Ballonkatheter-Stent-vorrichtung nach einem der vorangehenden Ansprüche, wobei der Stent (5; 5') eine reguläre Gitterstruktur aus Gitterstabelementen aufweist und die Faltwerke (7; 11) mindestens teilweise von Gitterstabelementen (9a, 9b; 13a, 13b) gebildet oder an diese fest angefügt sind.

7. Ballonkatheter-Stent-Vorrichtung nach einem der vorangehenden Ansprüche, wobei Endabschnitte von Faltkanten der Faltwerke (7; 11) mit Querschnittsverjüngungen oder Perforationen als Soll-Biegestellen oder einem Formgedächtnis-Material gebildet sind.

8. Ballonkatheter-Stent-Vorrichtung nach einem der vorangehenden Ansprüche, wobei mindestens ein Teil der Faltwerke (7; 11) jeweils mindestens eine Schneid- oder Sägekante zum schneidenden oder sägenden Eingriff in ein äußeres Material, insbesondere eine Stenose eines Blutgefäßes, aufweist.

9. Ballonkatheter-Stent-Vorrichtung nach einem der vorangehenden Ansprüche, wobei mindestens ein Teil der Faltwerke (7; 11) jeweils mindestens eine gebogene, insbesondere konvex gekrümmte, Fixierungskante zum Eingriff in eine äußere Struktur, insbesondere die Wandung eines Blutgefäßes oder Organs, aufweist.

10. Ballonkatheter-Stent-Vorrichtung nach einem der vorangehenden Ansprüche, wobei auf der Umfangsfläche des Stents (5; 5') mindestens in Zwischenbereichen zwischen einigen Faltwerken (7; 11) elastisch oder plastisch verformbare Ausgleichsbereiche zur Verringerung der resultierenden Verkürzung des Stents vorgesehen sind.

11. Ballonkatheter-Stent-Vorrichtung nach einem der vorangehenden Ansprüche, wobei die Faltwerke (7; 11) integral mit der übrigen Struktur des Stents (5; 5') und insbesondere mittels des gleichen Formgebungsverfahrens wie jene gebildet sind.

12. Ballonkatheter-Stent-Vorrichtung nach einem der Ansprüche 1 bis 10, wobei die Faltwerke (7; 11) aus einem anderen Material als die übrige Struktur des Stents (5; 5') gebildet und in einem gesonderten Anfügungs-Schritt an jene angefügt sind.

13. Ballonkatheter-Stent-Vorrichtung nach einem der vorangehenden Ansprüche, wobei der Stent (5; 5') und insbesondere auch die Faltwerke (7; 11) mindestens abschnittsweise aus einem Formgedächtnis-Material bestehen.

14. Ballonkatheter-Stent-Vorrichtung nach einem der vorangehenden Ansprüche, wobei der Stent (5; 5') mit den Faltwerken (7; 11) mindestens abschnittsweise mit dem Ballonkatheter (1; 1'), also dem Ballonabschnitt (3b; 3b') und/oder einem Ballonhals oder dem Katheterschlauch (3a), verbunden ist.
